Europäisches Patentamt

European Patent Office

Office européen des brevets

(11) Publication number: **0 664 113 A2**

## EUROPEAN PATENT APPLICATION

(21) Application number: **94120835.7**

(22) Date of filing: **28.12.94**

(51) Int. Cl.6: **A61K 7/06**

(30) Priority: **28.12.93 JP 335784/93**

(43) Date of publication of application:
**26.07.95 Bulletin 95/30**

(84) Designated Contracting States:
**DE GB NL**

(71) Applicant: **KAO CORPORATION**
**14-10, Nihonbashi,**
**Kayabacho 1-chome**
**Chuo-ku,**
**Tokyo (JP)**

(72) Inventor: **Muraoka, Tsutomu**
**4-12-201, Tachibana 5-chome**
**Sumida-ku,**
**Tokyo (JP)**

(74) Representative: **Hansen, Bernd, Dr.**
**Dipl.-Chem. et al**
**Hoffmann, Eitle & Partner,**
**Patentanwälte,**
**Arabellastrasse 4**
**D-81925 München (DE)**

(54) **Hairdressing composition.**

(57) A hairdressing composition is disclosed, which comprises from 0.1 to 10 % by weight of a water-dispersible polyester resin; from 0.01 to 10 % by weight of a thickening agent; and water. The hairdressing composition is excellent in the hairdressing performance, facilitates hairstyling, gives a less sticky and good touch and imparts an excellent finishing feel and a long-lasting style retention.

EP 0 664 113 A2

FIELD OF THE INVENTION

This invention relates to a hairdressing composition which exhibits a particularly excellent hairstyling performance in a styling process and has a good touch and a long-lasting style retention property.

BACKGROUND OF THE INVENTION

Hairstyling agents are roughly classified into processing agents which are used in the course of hairstyling and finishing agents which are used after the completion of hairstyling. The finishing agent, which is used in order to retain the hairstyle, should be provided in a form that can be applied to the hair without dishevelling the finished hairstyle. In addition, it should quickly dry up after the application, whereby the hairstyle is fixed. In contrast, the processing agent is applied to the hair in order to set the hair into the desired style with the use of a brush, a comb or, in some cases, a dryer. Therefore, it should have an excellent setting (hairdressing) performance.

In order to improve the hairdressing performance, sticky oils and polymers such as polyoxypropylene butyl ether and polyoxypropylene butyl ether phosphate have been incorporated in hairstyling agents. However, although these sticky components exhibit a certain degree of the hairdressing effect, they seriously deteriorate combing or brushing property of the hair in the course of hairstyling, which results in poor facility in hairdressing. In addition, the hair becomes highly sticky during or after styling, which brings about another problem in touch.

Therefore, attempts have been made to reduce the content of the sticky oil or polymer or to add a less sticky component such as silicone. In these cases, however, there arises another problem of deterioration in the hairdressing performance or the hairstyle retention performance after finishing.

Accordingly, there has been urgently required to develop a hairdressing composition which is excellent in the hairdressing performance, facilitates hairdressing and gives a good touch and a long-lasting hairstyle retention.

SUMMARY OF THE INVENTION

Under these circumstances, the present inventors have conducted extensive studies. As a result, they have successfully found that a hairdressing composition which is excellent in the hairdressing performance in a styling process, facilitates hairstyling and gives a good touch with less stickiness and a long-lasting hairstyle retention can be obtained by employing a combination of a water-dispersible polyester resin with a thickening agent. The present invention has been thus completed.

Accordingly, the present invention provides a hairdressing composition comprising:
(A) from 0.1 to 10 % by weight of a water-dispersible polyester resin;
(B) form 0.01 to 10 % by weight of a thickening agent; and
(C) water.

DETAILED DESCRIPTION OF THE INVENTION

The water-dispersible polyester resin to be used as component (A) in the present invention may be arbitrarily selected without restriction, so long as it is insoluble but dispersible in water. Typical and preferred example thereof include a polyester having $-SO_3M$ groups, wherein M represents a hydrogen atom or a metal ion, in a sufficient amount for the dispersion in water.

Preferred examples of the above-mentioned polyester having $-SO_3M$ groups include those disclosed in U.S. Patent 5,218,042, hereby incorporated by reference, which are obtained by condensing at least one dicarboxylic acid, at least one diol and at least one bifunctional aromatic monomer having at least one sulfonate group on its aromatic nucleus.

The dicarboxylic acid to be used herein may be any of aliphatic dicarboxylic acids, alicyclic dicarboxylic acids and aromatic dicarboxylic acids. Examples thereof include oxalic acid, malonic acid, dimethylmalonic acid, succinic acid, glutaric acid, adipic acid, trimethyladipic acid, pimelic acid, 2,2-dimethylglutaric acid, azelaic acid, sebacic acid, fumaric acid, maleic acid, itaconic acid, phthalic acid, dodecanedioic acid, 1,3-cyclopentanedicarboxylic acid, 1,2-cyclohexanedicarboxylic acid, 1,3-cyclohexanedicarboxylic acid, 1,4-cyclohexanedicarboxylic acid, phthalic acid, terephthalic acid, isophthalic acid, 2,5-norbornenedicarboxylic acid, 1,4-naphthalic acid, diphenic acid, 4,4'-oxydibenzoic acid, diglycolic acid, thiodipropionic acid, 4,4'-sulfonyldibenzoic acid, 2,5-naphthalenedicarboxylic acid and 2,6-naphthalenedicarboxylic acid. Either one of these dicarboxylic acids or a combination of two or more of them may be used.

Among these dicarboxylic acids, isophthalic acid, terephthalic acid and phthalic acid are particularly preferred.

Examples of the diol include ethylene glycol, propylene glycol, 1,3-propanediol, 2,4-dimethyl-2-ethylhexane-1,3-diol, 2,2-dimethyl-1,3-propanediol, 2-ethyl-2-butyl-1,3-propanediol, 2-ethyl-2-isobutyl-1,3-propanediol, 1,3-butanediol, 1,4-butanediol, 1,5-pentanediol, 1,6-hexanediol, 2,2,4-trimethyl-1,6-hexanediol, thiodiethanol, 1,2-cyclohexanedimethanol, 1,3-cyclohexanedimethanol, 1,4-cyclohexanedimethanol, 2,2,4,4-tetramethyl-1,3-cyclobutanediol, p-xylylenediol, diethylene glycol, triethylene glycol, tetraethylene glycol, pentaethylene glycol, hexaethylene glycol, heptaethylene glycol, octaethylene glycol, nonaethylene glycol, decaethylene glycol, hydroxypivalyl hydroxypivalate, dipropylene glycol, 1,10-decanediol, 1,3-butanediol, bisphenol A hydride, 1,4-butanediol and neopentyl glycol. Either one of these diols or a combination of two or more of them may be used.

Among these diols, diethylene glycol, 1,2-, 1,3- and 1,4-cyclohexanedimethanols, triethylene glycol, ethylene glycol, 1,3-propanediol and 1,4-butanediol are particularly preferred.

The bifunctional aromatic monomer having at least one sulfonate group on its aromatic nucleus (hereinafter referred to as a "bifunctional monomer having sulfonate group(s)") to be used in the present invention is an aromatic diol or aromatic dicarboxylic acid having at least one sulfonate group on its aromatic nucleus. Preferred examples thereof include aromatic dicarboxylic acids and derivatives thereof and aromatic diols, each having at least one -$SO_3M$ group. Examples of the metallic ion represented by M of -$SO_3M$ include $Na^+$, $Li^+$, $K^+$, $Mg^{2+}$, $Ca^{2+}$, $Cu^{2+}$, $Fe^{2+}$ and $F^{3+}$, of which $Na^+$, $Li^+$ and $K^+$ being preferred.

Examples of the aromatic nucleus to which the -$SO_3M$ group is bound include benzene, naphthalene, anthracene, diphenyl, oxydiphenyl, sulfonyldiphenyl and methylenediphenyl.

Among these bifunctional monomers having sulfonate group(s), sulfoisophthalic acid, sulfoterephthalic acid, sulfophthalic acid, 4-sulfonaphthalene-2,7-dicarboxylic acid, derivatives of these acids and sodium salts thereof are preferred, and sulfoisophthalic acid and sulfoterephthalic acid are particularly preferred.

Either one of these bifunctional monomers having sulfonate group(s) or a combination of two or more of them may be used.

The condensation reaction of the dicarboxylic acid, the diol and the bifunctional monomer having sulfonate group(s) may be carried out in accordance with the conventional method. For example, a water-dispersible polyester can be obtained in a dehydration reaction of a mixture of these monomers in the presence of a catalyst such as dibutyltin oxide at a temperature of about 200°C.

Among these water-dispersible polyester resins, one obtained by condensing diethylene glycol, cyclohexanedimethanol, isophthalic acid and sulfoisophthalic acid is particularly preferable. This type of the water-dispersible polyester resin is also available as marketed products, for example, Eastman AQ 38S and 55S (manufactured by Eastman Chemical Products Inc.).

Either one of these water-dispersible polyester resins or a combination of two or more of them may be used in the present invention. The content of the water-dispersible polyester resin in the hairdressing composition ranges from 0.1 to 10 % by weight, preferably from 0.5 to 8 % by weight, and more preferably from 1 to 6 % by weight, based on the total weight of the composition. When the content is smaller than 0.1 % by weight, the styling (setting) performance and the style retention property tend to deteriorate. On the other hand, when the content exceeds 10 % by weight, the styling performance (workabillty) and the feel of the finishing tend to deteriorate.

Examples of the thickening agent to be used as component (B) include polysaccharides, polysaccharide derivatives and polyvinyl alcohols. It is preferable to use a thickening agent having a viscosity of 20 cP or above at 25°C when formulated into a 5 % aqueous solution.

Specific examples of the polysaccharide include those originating in plants, those originating in microorganisms and those originating in animals. Among them, carrageenan, locust bean gum, guar gum, gum arabic, gum tragacanth, pectin and xanthan gum are preferred.

Specific examples of the polysaccharide derivative include cellulose derivatives, starch derivatives and alginic acid derivatives as disclosed, for example, in "Polymer and Thickeners for Cosmetics", Cosmetics & Toiletries Vol. 108, 95 (1993), hereby incorporated by reference, such as methyl cellulose, ethyl cellulose, hydroxyethyl cellulose, methylhydroxypropyl cellulose, cetylhydroxyethyl cellulose, cocodimonium hydroxypropyl oxyethyl cellulose, hydroxypropylmethyl cellulose, hydroxypropyl cellulose, nonoxynyl hydroxyethyl cellulose, alkyl-modified hydroxyethyl cellulose quaternary ammonium salt (POLYQUATERNIUM-24), steayldimonium hydroxyethyl cellulose, stearyldimonium hydroxypropyl oxyethyl cellulose, guar hydroxypropyltrimonium chloride, hydroxypropyl guar, and a mixture of a polymeric quaternary ammonium salt of hydroxyethyl cellulose and a trimethyl ammonium substituted epoxide (POLYQUATERNIUM-10). Among them, methyl cellulose, ethyl cellulose, hydroxyethyl cellulose and methylhydroxypropyl cellulose are preferred.

Specific and preferred example of the polyvinyl alcohol include one having a saponification value of 70 or more, preferably 85 or more.

Either one of these thickening agents or a combination of two or more of them may be used.

The viscosity of the final product of the hairdressing composition of the present invention increases as the content of the thickening agent increases. The content of the thickening agent in the hairdressing composition ranges from 0.01 to 10 % by weight, preferably from 0.05 to 5 % by weight, and more preferably from 0.1 to 3 % by weight. When the content is smaller than 0.01 % by weight, the styling (setting) performance and the feel of the finishing tend to deteriorate. On the other hand, when the content exceeds 10 % by weight, the styling performance (workability) and the style retention tend to deteriorate.

In the hairdressing composition of the present invention, it is preferred that components (A) and (B) are present in a weight ratio of component (A) to component (B) of from 0.1 to 100, more preferably from 0.1 to 50, and still more preferably from 0.1 to 25.

In addition to the above-mentioned components, the hairdressing composition of the present invention may contain surfactants. Any of anionic, nonionic, ampholytic and cationic surfactants may be used. Among all, nonionic surfactants are particularly preferred. Examples thereof include polyoxyalkylene-added alkyl ethers such as polyoxyethylene cetyl ether, polyoxyethylene stearyl ether, polyoxypropylene cetyl ether and polyoxyethylene oxypropylene cetyl ether.

The surfactant, if employed, may be contained in the hairdressing composition in an amount of from 0.01 to 5 % by weight based on the total weight of the composition.

The hairdressing composition of the present invention may further contain components which are generally used as materials for producing cosmetics, for example, glycerides such as castor oil, cacao oil, mink oil, avocado oil and olive oil; waxes such as beeswax, whale wax, lanolin and carnauba wax; alcohols such as cetyl alcohol, oleyl alcohol, hexadecyl alcohol, lauryl alcohol, stearyl alcohol, isostearyl alcohol, 2-octyldodecanol, propylene glycol, polypropylene glycol, glycerol and ethanol; esters such as isopropyl myristate, isopropyl laurate, hexyl laurate, cetyl lactate, propylene glycol monostearate, oleyl oleate, hexadecyl 2-ethylhexanoate and octyldodecyl myristate; silicones such as dimethyl polysiloxane, polyether-modified silicone, amino-modified silicone and alkyl-modified silicone; parabens such as methylparaben and ethylparaben; perfumes; colorants; antioxidants; UV absorbers; and plant extracts, so long as the effects of the present invention are not deteriorated thereby.

The hairdressing composition of the present invention can be produced in accordance with the conventional method. It may be formulated into either aerosol or non-aerosol products, for example, a hair foam, a hair gel, a hair spray, a set lotion, a hair cream or a pump mist. Among these products, those being in a state of a foam, a lotion or a gel are preferred and a styling agent for a hairstyling process is more preferred.

When the hairdressing composition of the present invention is to be formulated into an aerosol, it is enclosed in a sealed pressure container together with a propellant. As the propellant, those employed in the art such as a liquefied gas or a compressed gas may be used. Specific examples thereof include dimethyl ether, liquefied petroleum gas, nitrogen gas and carbon dioxide gas.

When the hairdressing composition of the present invention is to be formulated into a foam product, liquefied petroleum gas or dimethyl ether is preferred as the propellant and it is contained in the composition in an amount of, preferably from 3 to 50 % by weight, and more preferably from 5 to 25 % by weight, based on the total weight of the composition.

In view of facility in the styling (setting performance and workability), it is preferred that the final product or a stock solution of an aerosol product of the hairdressing composition of the present invention has a viscosity at 25°C of at least 20 cP, more preferably at least 50 cP, and furthermore preferably at least 100 cP.

The term "viscosity" as used herein means the value measured at 25°C by means of a BM type rotary viscometer (manufactured by Tokyo Keiki) at 30 rpm.

The hairdressing composition of the present invention is excellent particularly in the hairdressing performance in a styling process, facilitates hairstyling, gives a pleasant touch with less stickiness and imparts a good feel of the finishing and a long-lasting hairstyle retention.

To further illustrate the present invention in greater detail, and not by way of limitation, the following Examples will be given.

EXAMPLE 1

Lotion type hairdressing compositions of the formula shown in Table 1 were produced in the conventional method. Each product was evaluated for its facility in brushing, stickiness, facility in styling

4

and hair gathering property during the styling process and its stickiness and style retention property after the finishing. Further, the viscosity of the product at 25°C was measured. Table 1 summarizes the results.

(Evaluation method)

(1) Facility in brushing, facility in styling, hair gathering property and stickiness during and after hairdressing:

A wig of a medium length was washed with a standard shampoo and dried with a towel. Thereafter, 5 g of a lotion type hairdressing composition was applied to the moist hair. Then, 10 female panelists skilled in the art dressed the hair with the use of a dryer and a brush, and organoleptically evaluated each item of the performance in accordance with the following criteria.

Facility in Brushing (Brushing)

A: very easy
B: easy
C: difficult
D: very difficult

Facility in Styling (Styling)

A: very easy
B: easy
C: difficult
D: very difficult

Hair Gathering property (Gathering)

A: very easy
B: easy
C: difficult
D: very difficult

Stickiness

A: not sticky
B: somewhat sticky but not offensive
C: sticky and offensive
D: seriously sticky

(2) Style retention property:

A hair bundle of 18 cm in length and 5 g in weight was washed with a standard shampoo and dried with a towel. Then, 0.2 g of a lotion type hairdressing composition was applied to this hair bundle. Next, it was wound around a rod of 2 cm in diameter and then dried in a room at 25°C and 65 % RH for 24 hours. After drying, the hair bundle was taken off from the rod and set in a vibrator. After vibrating at 120 stroke/min at 20°C under 98 % RH for 30 minutes, the elongation of the hair curl was observed and the style retention property was determined by the following equation:

Style retention = $(L - L_1)/(L - L_0) \times 100$

wherein L means the initial length of the hair bundle (18 cm); $L_0$ means the hair bundle length after taking off the rod; and $L_1$: the hair bundle length after vibrating for 30 min.

The results are expressed in accordance with the criteria as will be shown below.

5

(Criteria for evaluation)

A: 80 % or more
B: 60 or more to less than 80 %
C: 20 or more to less than 60 %
D: less than 20 %

EP 0 664 113 A2

TABLE 1

| Component (% by weight): | Product of the Invention | | | Comparative product | | | |
|---|---|---|---|---|---|---|---|
| | 1 | 2 | 3 | 1 | 2 | 3 | 4 |
| Water-dispersible polyester[*1] | 3.0 | 3.0 | 3.0 | 3.0 | – | 3.0 | 3.0 |
| Carrageenan[*2] | 0.1 | 0.7 | 1.5 | – | 1.5 | – | – |
| Vinyl copolymer[*3] | – | – | – | – | – | 1.0 | – |
| Polyvinylpyrrolidone[*4] | – | – | – | – | – | 0.5 | – |
| Acrylic copolymer[*5] | – | – | – | – | – | – | 1.5 |
| Aminomethylpropanol | – | – | – | – | – | – | 0.46 |
| Propylene glycol | 3.0 | 3.0 | 3.0 | 3.0 | 3.0 | 3.0 | 3.0 |
| Methylparaben | 0.1 | 0.1 | 0.1 | 0.1 | 0.1 | 0.1 | 0.1 |
| Purified water | balance | balance | balance | balance | balance | balance | balance |
| Evaluation: | | | | | | | |
| During styling: Brushing | B | A | A | D | C | D | C |
| Stickiness | B | B | B | C | B | D | D |
| Styling | B | A | A | C | C | C | C |
| Gathering | B | A | A | C | C | B | C |
| After finishing: Stickiness | B | B | B | C | B | D | C |
| Style retention | B | A | A | A | C | B | A |
| Viscosity at 25°C (cP) | 18 | 29 | 58 | 12 | 62 | – | – |

Notes:  *1:  Eastman AQ38, manufactured by Eastman Chemical
            Products Inc.

        *2:  Soageenan MV220, manufactured by Mitsubishi
            Rayon Co., Ltd.

        *3:  Polyvinylpyrrolidone/vinyl acetate copolymer;
            Luviskol VA 73W, manufactured by BASF AG

        *4:  K-90, manufactured by ISP Co.

        *5:  Octylacrylamide/acrylates/butylaminoethyl
            methacrylate copolymer; AMPHOMER LV-71,
            manufactured by National Starch Inc..

As the results shown in Table 1 clearly demonstrate, each of the hairdressing compositions according to the present invention is excellent in the hairdressing performance and the post-finishing performance.

EXAMPLE 2

Foam type hairdressing compositions of the formula shown in Table 2 were produced by the conventional method. Each product was evaluated for its facility in brushing, stickiness, facility in styling and hair gathering property during the styling process and the stickiness and style retention property after the finishing by the same methods as in the above Example 1. Further, the viscosity of the stock solution of the product (i.e., the composition free from the aerosol gas component (liquefied petroleum gas)) at 25°C was determined. Table 2 summarizes the results.

<u>TABLE 2</u>

| Component (% by weight): | Product of the Invention | | | |
|---|---|---|---|---|
| | 4 | 5 | 6 | 7 |
| Water-dispersible polyester (Eastman AQ38, manufactured by Eastman Chemical Products Inc.) | 5.0 | 5.0 | 5.0 | 5.0 |
| Carrageenan (Soageenan LX22, manufactured by Mitsubishi Rayon Co., Ltd.) | 3.0 | – | – | – |
| Xanthan gum (Echo Gum (KELTROL T), manufactured by Dainippon Pharmaceutical Co., Ltd.) | – | 0.8 | – | – |
| Hydroxyethyl cellulose (HEC Daicel SE550, manufactured by Daicel Chemical Industries, Ltd.) | – | – | 0.8 | – |
| Polyvinyl alcohol (GOHSENOL EG-40, manufactured by The Nippon Synthetic Chemical Industry, Co., Ltd.) | – | – | – | 0.8 |
| Polyoxyethylene (40 mol) cetyl ether | 0.3 | 0.3 | 0.3 | 0.3 |
| Propylene glycol | 5.0 | 5.0 | 5.0 | 5.0 |
| Polyether-modified silicone (Silicone KF353A, manufactured by Shin-Etsu Chemical Co., Ltd.) | 0.1 | 0.1 | 0.1 | 0.1 |
| Methylparaben | 0.1 | 0.1 | 0.1 | 0.1 |
| Perfume | 0.02 | 0.02 | 0.02 | 0.02 |
| Purified water | balance | balance | balance | balance |
| Liquefied petroleum gas | 10.0 | 10.0 | 10.0 | 10.0 |
| **Evaluation:** | | | | |
| In styling: Brushing | A | A | A | B |
| Stickiness | B | B | B | B |
| Styling | B | B | B | B |
| Gathering | A | A | A | B |
| After finishing: Stickiness | A | A | A | A |
| Style retention | A | A | A | B |
| Viscosity of stock solution at 25°C (cP) | 2852 | 880 | 75 | 16 |

As the results shown in Table 3 clearly demonstrated, each of the hairdressing compositions of the present invention is excellent in the hairdressing performance and the post-finishing performance.

EXAMPLE 3

A gel type hairdressing composition of the formula specified below was produced by the conventional method.

Then, the resulting gel hairdressing composition was evaluated in the same manner as in Example 1. As a result, it was found that it is excellent in the hairdressing performance and the post-finishing performance. The viscosity of this product at 25°C exceeded 20,000 cP.

| Component | Amount (% by weight) |
|---|---|
| Water-dispersible polyester (Eastman AQ55, manufactured by Eastman Chemical Products Inc.) | 5.0 |
| Hydroxyethyl cellulose | 1.0 |
| Carboxyvinyl polymer (Carbopol 940, manufactured by The B.F. Goodrich Co.) | 0.5 |
| Glycerol | 5.0 |
| Triethanolamine | to regulate to pH 7.5 |
| Methylparaben | 0.1 |
| Purified water | balance |

While the invention has been described in detail and with reference to specific examples thereof, it will be apparent to one skilled in the art that various changes and modifications can be made therein without departing from the spirit and scope thereof.

## Claims

1. A hairdressing composition comprising:
   (A) from 0.1 to 10 % by weight of a water-dispersible polyester resin;
   (B) form 0.01 to 10 % by weight of a thickening agent selected from polysaccharides, polysaccharide derivatives and polyvinyl alcohol; and
   (C) water.

2. The hairdressing composition of claim 1, wherein said water-dispersible polyester resin is one obtained by condensing at least one dicarboxylic acid, at least one diol and at least one bifunctional aromatic monomer having at least one sulfonate group on its aromatic nucleus.

3. The hairdressing composition of claim 1, wherein said water-dispersible polyester resin is one obtained by condensing diethylene glycol, cyclohexanedimethanol, isophthalic acid and sulfoisophthalic acid.

4. The hairdressing composition of claim 1, wherein said thickening agent is selected from carrageenan, locust bean gum, guar gum, gum arabic, gum tragacanth, pectin, xanthan gum, methyl cellulose, ethyl cellulose, hydroxyethyl cellulose, methylhydroxypropyl cellulose, and polyvinyl alcohol.

5. The hairdressing composition of claim 1, which has a viscosity of 20 cP or above at 25°C.

6. The hairdressing composition of claim 1, wherein component (A) and component (B) are present in a weight ratio of component (A) to component (B) of from 0.1 to 100.